# DEMANDE DE BREVET EUROPEEN

(11) **EP 0 776 662 A1**
(43) Date de publication de la demande: **04.06.1997**
(21) Numéro de dépôt: 96402609.0
(22) Date de dépôt: 02.12.1996
(51) Int. Cl.: A61K 31/54, A61K 9/00, A61K 47/40, A61K 47/48

(54) **Compositions pharmaceutiques à base de méquitazine**

(30) Priorité: 06.12.1995 FR 9514425
(71) Demandeur: Laboratoire Chauvin S.A., 34009 Montpellier Cédex 1 (FR)
(72) Inventeur: Coquelet, Claude, 34980 Saint Gely sur Fesc (FR); Latour, Elisabeth, 34000 Montpellier (FR); Maurin, Florence, 34570 Vailhauques (FR)
(74) Mandataire: Le Guen, Gérard

(57) **Abrégé**

Cette invention a pour objet un collyre prêt à l'emploi destiné au traitement des allergies oculaires, comprenant en solution aqueuse de la méquitazine et une β ou une γ-cyclodextrine éventuellement éthérifiée par des groupes alkyle en C₁-C₄ ou hydroxyalkyle en C₁-C₄, ladite cyclodextrine étant présente en un rapport au moins molaire vis à vis de la méquitazine.

## Description

La présente invention concerne des compositions pharmaceutiques à base de méquitazine.

La méquitazine est un dérivé de phénothiazine (10-(3-quinuclidinylmethyl)-phenothiazine) qui a été décrit comme antihistaminique H1 et qui est utilisé par voie orale pour le traitement de manifestations allergiques diverses, y compris au niveau oculaire.

Toutefois, la méquitazine a été décrite comme ayant une activité anesthésique légèrement supérieure à celle de la lidocaine (Hojo et al, Folia Pharmacologica Japonica 78(5), 403, 1981).

Par ailleurs, dans JP-A-02 003 610, on a décrit un sirop qui contient de la méquitazine (0,03 à 0,05 %), 40 à 60 % (p/v) d'un sucre tel que le sorbitol ou le mannitol et une β ou γ-cyclodextrine comme solubilisant.

La présente invention vise à fournir une composition destinée au traitement local des allergies oculaires qui présente une excellente tolérance et qui en particulier n'induit pas localement d'effet anesthésique, effet qui doit être évité dans un traitement local car il entraîne une diminution de la sensibilité cornéenne et donc des risques de dégradation de la cornée par suite d'un frottement incontrôlé par le patient.

La présente invention a pour objet un collyre prêt à l'emploi destiné au traitement des allergies oculaires, comprenant en solution aqueuse de la méquitazine et une β ou une γ-cyclodextrine éventuellement éthérifiée par des groupes alkyle en C₁-C₄ ou hydroxyalkyle en C₁-C₄, ladite cyclodextrine étant présente en un rapport au moins molaire vis à vis de la méquitazine.

Les β et γ cyclodextrines éventuellement éthérifiées sont des composés largement connus et commercialisés. Il s'agit de structures cycliques constituées respectivement par 7 ou 8 motifs d'anhydroglucose. Chacun des motifs glucose contient 3 groupes hydroxy qui peuvent être en partie éthérifiés par des groupes alkyle en C₁-C₄ ou hydroxyalkyle en C₁-C₄. Le degré de substitution par motif d'anhydroglucose varie généralement de 0,05 à 2, notamment de 0,2 à 2.

Dans la présente invention, on préfère tout particulièrement l'hydroxypropyl-β-cyclodextrine, mais on peut également utiliser en particulier l'hydroxyethyl-β-cyclodextrine, l'hydroxybutyl-β-cyclodextrine et l'hydroxypropyl-γ-cyclodextrine.

L'hydroxypropyl-β-cyclodextrine a avantageusement un taux d'éthérification de 0,4.

Le collyre selon la présente invention comprend avantageusement de 0,01 à 0,5 % en poids de méquitazine et notamment 0,05 % en poids de méquitazine.

L'hydroxypropyl-β-cyclodextrine est utilisée avantageusement dans un rapport molaire hydroxypropyl-β-cyclodextrine/méquitazine d'au moins 4. Un collyre préféré contient 0,05 % en poids de méquitazine et 1 % en poids d'hydroxypropyl-β-cyclodextrine.

Le collyre selon l'invention peut contenir en plus de la méquitazine, de la cyclodextrine et de l'eau, des adjuvants habituels pour un collyre, à savoir des conservateurs, des tampons et/ou des agents d'ajustement du pH et des agents d'isotonicité tels que le sorbitol, le mannitol ou le glucose. Ces agents d'isotonicité peuvent être présents en des proportions de 0 à 6 % en poids.

On donnera ci-après un exemple de préparation d'un collyre selon l'invention.

### EXEMPLE 1

On prépare un collyre prêt à l'emploi ayant la composition suivante :

| | |
|---|---|
| Méquitazine | 0,05 g |
| Chlorure de benzalkonium (solution à usage ophtalmique) | 0,01 g |
| Edétate de sodium | 0,05 g |
| Hydroxypropyl-β-cyclodextrine * | 1,00 g |
| Acide borique | 1,50 g |
| Arginine | 0,46 g |
| Sorbitol à 70 % cristallisable | 1,70 g |
| Eau purifiée qsp | 100,00 ml |
| pH | 6,00 |

| | |
|---|---|
| * Produit commercialisé par Janssen sous la marque Encapsin HPB ayant une masse moléculaire moyenne de 1300, des substitutions en positions 1 et 4 avec un degré moyen de substitution de 0,4. | |

Le collyre est préparé par dissolution dans l'eau de l'édétate de sodium, de l'acide borique puis de l'hydroxypropylcyclodextrine.

On ajoute ensuite la méquitazine puis le sorbitol, l'arginine pour ajuster le pH à 6 puis le chlorure de benzalkonium.

On donnera ci-après des résultats des études pharmacologiques mettant en évidence les propriétés du collyre selon l'invention.

1. Etude de l'activité antihistaminique de collyres contenant de la méquitazine avec ou sans hydroxypropyl-β-cyclodextrine dans un modèle d'allergie oculaire induite à l'histamine chez le cobaye.

Les expériences ont été réalisées sur des cobayes Hartley mâles, d'un poids moyen de 300 g, provenant du centre d'élevage Charles River France (Saint Aubin les Elbeuf - 76140 CLEON), acclimatés pendant cinq jours dans l'animalerie avant le début de l'étude.

Les animaux, anesthésiés à la kétamine (40 mg/kg par voie intramusculaire) et à la xylazine (5 mg/kg par voie sous-cutanée), ont été rasés au niveau de la région superosternale et une incision de 3 cm a été pratiquée afin de dégager une veine jugulaire.

Une solution de Bleu Evans à 4 % dans du sérum physiologique a été injectée à raison de 0,5 ml/kg (20 mg/kg) dans la jugulaire à l'aide d'une seringue munie d'une aiguille 26 Gauge. Une pression de quelques secondes sur la veine permet la formation d'un caillot et évite toute hémorragie.

Une minute après injection de la solution de Bleu Evans, l'oedème conjonctival a été induit dans les deux yeux par instillation de 10 µl d'une solution contenant 30 µg d'histamine base dans du sérum physiologique.

Quinze minutes plus tard, l'animal a été sacrifié par injection intracardiaque de pentobarbital, et les paupières et le globe oculaire ont été prélevés immédiatement.

Ces prélèvements (après ouverture du globe et élimination du cristallin) ont été extraits pour chaque oeil séparément par 10 ml d'un mélange acétone/sulfate de sodium 0,5 % (7v/3v) pendant une nuit sous agitation douce. Après centrifugation à 1800 x g pendant 15 minutes, la concentration de Bleu Evans dans l'extrait a été déterminée par spectrophotométrie à 620 nm, comparativement à une courbe d'étalonnage.

Les expériences ont été réalisées sur 54 animaux répartis en 9 lots de six animaux, traités par les différents collyres suivants :
- sérum physiologique (lot témoin),
- collyres contenant de la méquitazine à différentes concentrations, sans hydroxypropyl-β-cyclodextrine.

| | 0,1 % | 0,05 % | 0,005 % | 0,0005 % |
|---|---|---|---|---|
| Méquitazine | 0,10 g | 0,05 g | 0,005 g | 0,0005 g |
| Edétate de sodium | 0,05 g | 0,05 g | 0,05 g | 0,05 g |
| Arginine | 0,46 g | 0,46 g | 0,46 g | 0,46 g |
| Sorbitol à 70 % cristallisable | 1,70 g | 1,70 g | 1,70 g | 1,70 g |
| Acide borique | 1,50 g | 1,50 g | 1,50 g | 1,50 g |
| Chlorure de benzalkonium solution à usage ophtalmique | 0,01 g | 0,01 g | 0,01 g | 0,01 g |
| Eau purifiée qsp | 100 ml | 100 ml | 100 ml | 100 ml |
| pH | 6,0 ± 0,1 | | | |

- collyres contenant la méquitazine à différentes concentrations avec hydroxypropyl-β-cyclodextrine

| | 0,1 % | 0,05 % | 0,005 % | 0,0005 % |
|---|---|---|---|---|
| Méquitazine | 0,10 g | 0,05 g | 0,005 g | 0,0005 g |
| Edétate de sodium | 0,05 g | 0,05 g | 0,05 g | 0,05 g |
| Hydroxypropyl-β cyclodextrine | 2,00 g | 1,00 g | 0,10 g | 0,01 g |
| Arginine | 0,46 g | 0,46 g | 0,46 g | 0,46 g |
| Sorbitol à 70 % cristallisable | 1,70 g | 1,70 g | 1,70 g | 1,70 g |
| Acide borique | 1,50 g | 1,50 g | 1,50 g | 1,50 g |
| Chlorure de benzalkonium solution à usage ophtalmique | 0,01 g | 0,01 g | 0,01 g | 0,01 g |
| Eau purifiée qsp | 100 ml | 100 ml | 100 ml | 100 ml |
| pH | 6,0 ± 0,1 | | | |

Le rapport en poids hydroxypropyl-β-cyclodextrine/méquitazine a été maintenu constant.

Une heure avant l'induction de l'oedème conjonctival, les animaux ont été traités dans les deux yeux par instillation de 10 µl du collyre testé. Les résultats, exprimés sous forme de moyenne ± écart-type, sont résumés dans le tableau suivant :

| Collyre | Fuite vasculaire (µg de Bleu Evans/oeil) | Inhibition% |
|---|---|---|
| Sérum physiologique | 31,28 ± 11,42 | - |
| Méquitazine 0,1 % | 4,37 ± 4,44 | 86 |
| Méquitazine 0,1 % (HPβCD) | 4,16 ± 4,72 | 87 |
| Méquitazine 0,05 % | 2,73 ± 3,01 | 91 |
| Méquitazine 0,05 % (HPβCD) | 3,12 ± 3,07 | 90 |
| Méquitazine 0,005 % | 7,73 ± 6,83 | 75 |
| Méquitazine 0,005 % (HPβCD) | 6,97 ± 5,43 | 78 |
| Méquitazine 0,0005 % | 10,07 ± 5,48 | 68 |
| Méquitazine 0,0005 % (HPβCD) | 9,05 ± 3,35 | 71 |

Ces résultats montrent que la méquitazine administrée sous forme collyre inhibe l'augmentation de perméabilité vasculaire induite par l'histamine et que l'addition d'hydroxypropyl-β-cyclodextrine dans ces collyres, dans le rapport en poids de 20 pour 1, ne modifie pas l'activité antihistaminique de la méquitazine.

2. Effet de la méquitazine sous forme de collyre en présence ou en l'absence d'hydroxypropyl-β-cyclodextrine sur la sensibilité cornéenne chez le lapin.

L'objectif de cette étude était de comparer deux formulations de méquitazine en collyre à 0,2 % afin de vérifier si la présence d'hydroxypropyl-β-cyclodextrine dans l'excipient était susceptible de modifier l'activité anesthésique locale de ce principe actif.

Les expériences ont été réalisées sur des lapins mâles albinos Néo-zélandais pesant de 3 kg à 3,5 kg, provenant de l'élevage Charles River France (St Aubin les Elbeuf - 76410 CLEON), acclimatés pendant 5 jours minimum dans l'animalerie (température : 19 ± 2°C ; humidité relative : 55 ± 10 % ; éclairage : 12 heures d'éclairage artificiel - 12 heures de nuit).

On a utilisé pour les tests les compositions suivantes :
- Collyre contenant 0,2 % de méquitazine sans hydroxypropyl-β-cyclodextrine

| | Méquitazine | Placebo |
|---|---|---|
| Méquitazine | 0,20 g | - |
| Edétate de sodium | 0,05 g | 0,05 g |
| Arginine | qs pH6 | 0,07 g |
| Sorbitol à 70 % cristallisable | 6,79 g | 6,79 g |
| Acide chlorhydrique | qs dissolution | qs pH6 |
| Chlorure de benzalkonium solution à usage ophtalmique | 0,01 g | 0,01 g |
| Eau purifiée qsp | 100,00 ml | 100,00 ml |
| pH | 6,00 | 6,00 |

- Collyre contenant 0,2 % de méquitazine avec hydroxypropyl-β-cyclodextrine (Encapsin ® HPB)

| | Méquitazine | Placebo |
|---|---|---|
| Méquitazine | 0,20 g | - |
| Edétate de sodium | 0,05 g | 0,05 g |
| Hydroxypropyl-β-cyclodextrine | 4,00 g | 4,00 g |
| Arginine | qs pH6 | 0,07 g |
| Sorbitol à 70 % cristallisable | 5.83 g | 5,83 g |
| Acide chlorhydrique | qs dissolution | qs pH6 |
| Chlorure de benzalkonium solution à usage ophtalmique | 0,01 g | 0,01 g |
| Eau purifiée qsp | 100,00 ml | 100,00 ml |
| pH | 6,00 | 6,00 |

La sensibilité cornéenne a été mesurée au centre de la cornée avec l'esthésiomètre de Cochet et Bonnet (Luneau Ophtalmologie, Chartres).

Les animaux ont été préalablement sélectionnés en fonction de leur seuil de réponse (pression inférieure ou égale à 2,40 g/mm²) et répartis en deux lots de huit animaux.

Le jour de l'expérience, la sensibilité a été mesurée trois fois dans les deux yeux à intervalles de 20 minutes avant tout traitement (temps 0). Les animaux ont été traités dans un oeil par une instillation de 25 µl du collyre contenant la méquitazine à 0,2 %, formulée en présence ou en l'absence d'hydroxypropyl-β-cyclodextrine ; l'oeil controlatéral a reçu 25 µl du placebo correspondant. La sensibilité cornéenne a été déterminée dans les deux yeux 5, 10,20, 30, 45 et 60 minutes après instillation.

La sensibilité cornéenne est exprimée sous forme de pression exercée (en g/mm²); une augmentation de la pression correspond à une diminution de la sensibilité cornéenne. Les résultats sont exprimés sous forme de moyenne ± erreur standard.

Une analyse de variance factorielle a été utilisée suivie du test de comparaisons multiples de Newman-Keuls pour déterminer le niveau de signification de la différence entre les moyennes ; cette différence est considérée comme significative lorsque p est inférieure à 0,05 (*).

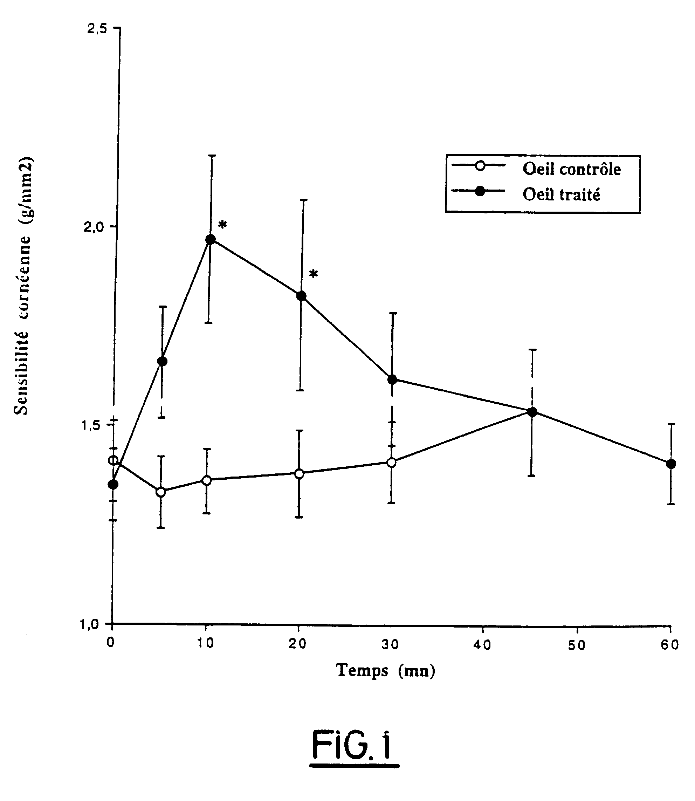

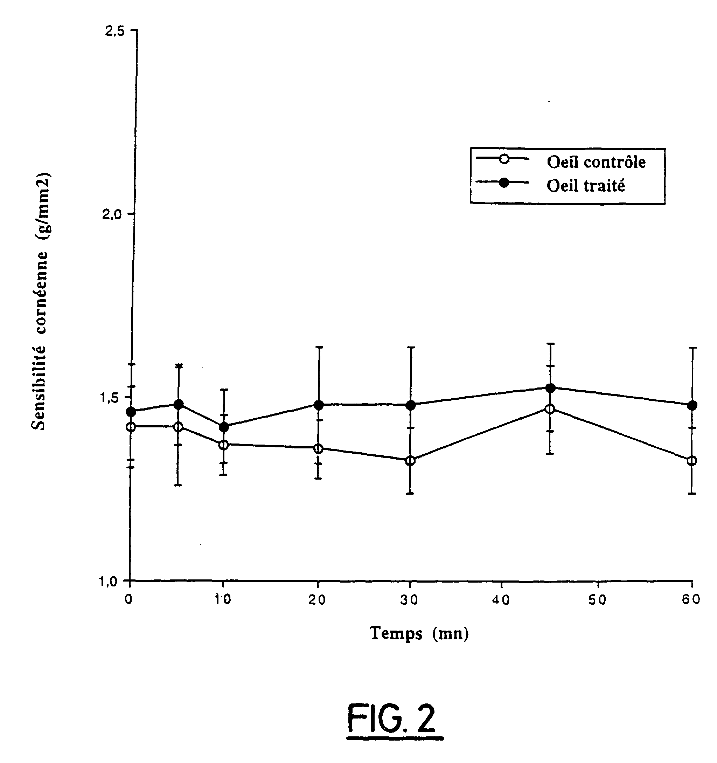

Les résultats obtenus avec un collyre sans HPβCD et avec HPβCD sont montrés respectivement sur les figures 1 et 2.

La méquitazine en collyre à 0,2 % formulée sans addition d'hydroxypropyl-β-cyclodextrine, provoque une diminution significative de la sensibilité cornéenne 10 et 20 minutes après administration d'une goutte, comparativement aux yeux controlatéraux traités par le placebo (figure 1).

Par contre, lorsque la méquitazine est administrée à la même concentration en présence d'hydroxypropyl-β-cyclodextrine, aucune hypoesthésie cornéenne significative n'est observée (figure 2).

L'addition d'hydroxypropyl-β-cyclodextrine dans l'excipient du collyre à la méquitazine entraîne donc une disparition des effets anesthésiques oculaires observés après instillation de ce collyre.

Au vu de l'étude 1 sur l'effet antihistaminique et de l'étude 2 sur l'effet anesthésique, il apparaît que, de manière surprenante, grâce à la présence d'hydroxypropyl-β-cyclodextrine, l'effet anesthésique est supprimé alors que l'effet antihistaminique n'est pas affecté.

## Revendications

1. Collyre prêt à l'emploi destiné au traitement des allergies oculaires, comprenant en solution aqueuse de la méquitazine et une β ou une γ-cyclodextrine éventuellement éthérifiée par des groupes alkyle en C₁-C₄ ou hydroxyalkyle en C₁-C₄, ladite cyclodextrine étant présente en un rapport au moins molaire vis à vis de la méquitazine.

2. Collyre selon la revendication 1, dans lequel la cyclodextrine est l'hydroxypropyl-β-cyclodextrine.

3. Collyre selon la revendication 2, dans lequel l'hydroxypropyl β-cyclodextrine a un degré de substitution par les groupes hydroxypropyle de 0,4.

4. Collyre selon l'une quelconque des revendications 1 à 3, comprenant de 0,01 à 0,5 % en poids de méquitazine.

5. Collyre selon l'une quelconque des revendications 1 à 4, dans lequel l'hydroxypropyl β-cyclodextrine est présente dans un rapport molaire d'au moins 4 vis à vis de la méquitazine.

6. Collyre selon l'une quelconque des revendications 1 à 3, comprenant 0,05 % en poids de méquitazine.

7. Collyre selon la revendication 6, comprenant 1 % en poids d'hydroxypropyl β-cyclodextrine.

8. Collyre selon l'une quelconque des revendications précédentes, comprenant de 0 à 6 % en poids d'un agent d'isotonicité.
